(19) Europäisches Patentamt
European Patent Office
Office européen des brevets

(11) Publication number: **0 266 047 A2**

# (12) EUROPEAN PATENT APPLICATION

(21) Application number: 87308212.7

(22) Date of filing: 16.09.87

(51) Int. Cl.[4]: C07C 2/28 , C07C 29/04 , C07C 41/01 , C07C 41/06 , C10L 1/02

(30) Priority: 26.09.86 GB 8623260

(43) Date of publication of application: 04.05.88 Bulletin 88/18

(84) Designated Contracting States: AT BE CH DE FR GB IT LI LU NL SE

(71) Applicant: The British Petroleum Company p.l.c.
Britannic House Moor Lane
London EC2Y 9BU(GB)

(72) Inventor: Gregory, Reginald
The British Petroleum Company p.l.c.
Chertsey Road
Sunbury-on-Thames Middlesex, TW16 7LN(GB)
Inventor: Hälsig, Claus-Peter
The British Petroleum Company p.l.c.
Chertsey Road
Sunbury-on-Thames Middlesex, TW16 7LN(GB)
Inventor: Smith, David John Harry
The British Petroleum Company p.l.c.
Chertsey Road
Sunbury-on-Thames Middlesex, TW16 7LN(GB)

(74) Representative: Crack, Richard David et al
BP INTERNATIONAL LIMITED Patents Division Chertsey Road
Sunbury-on-Thames Middlesex TW16 7LN(GB)

## (54) Process for the preparation of gasoline components from olefins.

(57) A process for the production of gasoline range products from $C_3$ to $C_6$ alkenes which comprises two steps:

(a) passing a feed containing the $C_3$ to $C_6$ alkenes and water in a molar ratio of alkene to water of from 3:1 to 90:1 over an acid hydration catalyst under hydration conditions to form a product containing oligomers of the alkenes, ethers and optionally alcohols and

(b) reacting alkenes containing branching at a carbon atom bearing a double bond with a $C_1$ to $C_6$ alcohol to form ethers.

The steps (a) and (b) can be performed in either order and can be followed by a third step (c) in which unreacted $C_3$ to $C_5$ alkenes are alkylated with isobutane in an alkylation step.

EP 0 266 047 A2

FIG.1

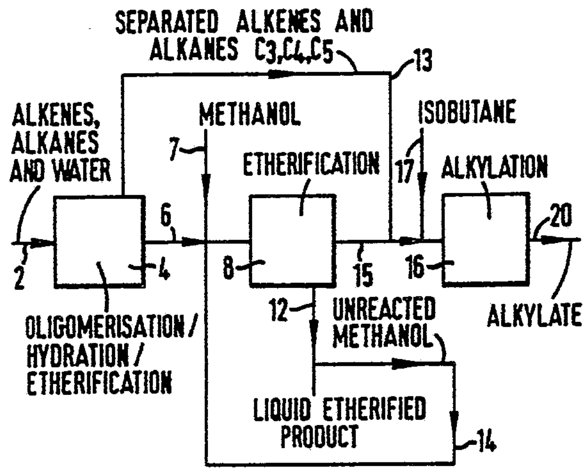


In one mode of operation.

| | | | |
|---|---|---|---|
| Stream (2) | 220,000 t/a | Stream (15) | |
| Water | 2,200 t/a | C4 | 154,500 t/a |
| Stream (6) | 67,700 t/a | Stream (17) | 72,800 t/a |
| C4 | 154,500 t/a | | Isobutane |
| Stream (7) Methanol | 9,026 t/a | Stream (20) | Alkylate 171,200 t/a |
| Stream (12) Liquid + Methanol | 76,726 t/a | | |

# PROCESS FOR THE PREPARATION OF GASOLINE COMPONENTS FROM OLEFINS

This invention relates to a process for the preparation of products suitable for addition to a motor gasoline which process comprises a combination of steps comprising (a) the hydration and oligomerisation of $C_3$ to $C_6$ alkenes, and (b) etherification of branched alkenes. The steps (a) and (b) can be performed in either order and optionally unreacted gas is alkylated in an alkylation step (c).

It has been previously proposed to incorporate oxygen-containing compounds into motor gasoline for a variety of different purposes. For example, to increase the amount of useful fuel, methanol has been described. To improve octane number, methyl tertiary butyl ether has been proposed. Oxygen-containing compounds have also been described for other purposes, for example, French Patent No 2149113 discloses the use of aliphatic ethers, optionally in combination with an alcohol, as a gasoline additive for reducing carbon monoxide emissions and isopropanol has been described as a carburettor deicing additive.

Our copending European Patent Application No 196902 discloses a process for the hydration and oligomerisation of one or more $C_3$ to $C_6$ olefins to produce a reaction product containing oligomers of the alkenes, ethers and optionally alcohols which comprises passing a feed containing $C_3$ to $C_6$ alkenes and water in a molar ratio of water to alkene of from 1:3 to 1:90 over an acid hydration catalyst under hydration conditions.

The present invention provides a process in which the hydration and oligomerisation process is combined with an etherification step to produce a further product suitable for use in a motor gasoline.

According to the present invention a process for the production of gasoline range products from $C_3$ to $C_6$ alkenes comprises the following steps.

(a) passing a feed containing $C_3$ to $C_6$ alkenes and water in a molar ratio of alkene to water of from 3:1 to 90:1 over an acid hydration catalyst under hydration conditions to form a product containing oligomers of the alkenes, ethers and optionally alcohols,

(b) an etherification step in which alkenes containing branching at a carbon atom bearing a double bond are reacted with a $C_1$ to $C_6$ alcohol to form ethers.

Whether step (a) precedes step (b) or visa versa the process can also include a third step, hereafter referred to as step (c) in which alkenes are reacted with isobutane (either added or already present) in an alkylation step to produce high octane alkylate.

Conveniently the process is performed so that step (a) precedes step (b) and the alkenes from step (a) are passed to step (b).

Conveniently the feed to step (b) contains at least 10% by wt of branched alkenes.

Conveniently step (b) is operated in a manner to increase the amount of branched ethers in by at least 10% by wt preferably by at least 25% by wt.

Unreacted alkenes can be separated from the liquid product of step (a) and passed to the alkylation step.

The step (a) can be carried out as described in our copending European Patent Application No 196902. The feed to the first step of the process whether step (a) or (b) can conveniently be provided by refinery process streams a substantial proportion of which comprise $C_3$ to $C_5$, more preferably $C_3$ and/or $C_4$, alkenes such as, for example, $C_3/C_4$ streams from catalytic cracking.

References to a feed containing $C_3$ to $C_6$ alkenes should not be taken to mean that only alkenes of this carbon number range are present. For example ethylene can be present in amounts up to 10% or 15% by weight of the feed.

The feed to the first step of the claimed process can also contain alkanes and many refinery streams such as a $C_3/C_4$ cut from a cat. cracker will be such a mixture. Nor is the term $C_3$ to $C_6$ alkenes intended to mean that alkenes of every carbon number from $C_3$ to $C_6$ will be present. The feed can contain one or more alkenes in this carbon number range. Most commonly available streams will be mixtures.

The feed to the first step can conveniently be a mixture of alkenes and alkanes of carbon number $C_3$ to $C_6$ and preferably contains at least 95% by weight of such compounds, more preferably at least 98%. More preferably the feed contains at least 95% by weight of alkenes and alkanes of carbon number $C_3$ to $C_5$.

The content of alkanes (including isoalkanes) can be up to 60% by weight or even 70% but since alkanes are unreactive in step (a) their content is preferably less than 50% by weight of the feed. The content of n-alkanes is preferably below 40% by weight, more preferably below 25%. Isobutane can be present and if present in an amount sufficient to provide a molar ratio of isobutane to alkenes of at least 1:2 in the feed to the alkylation step (c), it will not be necessary to add isobutane before step (c) although it may be preferred to do so in order to adjust the molar ratio to about 1:1. Typically isobutane can be present in an amount up to 60% by weight, although preferably it will not exceed 50% by weight.

It is preferred that the content of alkenes exceeds 50% more preferably 60%. Although there is no upper limit on the alkene content, refinery process streams consisting exclusively of alkenes are not usual, and the most commonly available feeds will comprise mixtures of alkenes and alkanes.

Particularly suitable feeds are those containing at least 95% of $C_3$ and $C_4$ alkenes and alkanes. Such a feed can contain from 20 to 85%, preferably 25 to 75% by weight of $C_3$ and $C_4$ alkenes and 5 to 65% by wt of $C_3$ and $C_4$ alkanes (including isoalkanes). The feed can comprise at least 95% by weight of $C_3$ or $C_4$ alkenes and alkanes. For example a feed containing at least 95% by wt of $C_4$ compounds con contain from 20 to 80% $C_4$ alkenes and 5 to 50% of $C_4$ alkanes (including isobutane).

For step (a) any conventional acid hydration catalyst may be used. Suitable catalysts include (A) cation-exchangeable layered clay in which there is a metal cation, (B) hydrogen ion-exchanged layered clays, (C) stabilised pillared interlayered clay and (D) cation exchanged organic resins. Catalysts of the type (A), (B) and (C) are known and are described, for example, in published European Patent Applications 0031687, 0031252, 0083970 and 0116469. Catalysts of type (D) are also known and some are commercially available.

Preferably the acid hydration catalyst is a cation exchange organic resin, more preferably one containing sulphonic acid groups.

Preferably the molar ratio of water to alkene in the feed is from 1:4 to 1:50 and more preferably in the case of a butene from 1:20 to 1:50 and in the case of a propene 1:5 to 1:20.

It has been found that the process of step (a) has unexpected advantages in that it can be controlled so as (i) to produce a liquid reaction product which contains at least 40% by weight of oligomers but less than 5% by wt of products of carbon number 12 and higher and also (ii) to produce a liquid reaction product containing less than 0.5% by weight of water, preferably less than 0.2%. This latter advantage is particularly significant in that the product can be added direct to a gasoline basestock without any intermediate drying step.

The process of step (a) is preferably operated with an alkene to water molar ratio of from 4:1 to 50:1, a temperature within the range 120 to 180°C, a pressure of from 20 to 100 bar absolute and an LHSV of feed from 0.5 to 10.

The reference to hydration conditions in step (a) is not intended to mean that hydration is the only reaction occurring in step (a). Other reactions take place such as oligomerisation and etherification.

The term oligomers is intended to include dimers.

The molar proportions of reactants and reaction conditions in step (a) can be selected so as (i) to produce a liquid reaction product containing from 70 to 99% by weight of oligomers, from 1 to 30% by weight of ethers and up to 10% by weight of alcohols or (ii) to produce a liquid reaction product containing from 40 to 70% by weight of oligomers, from 20 to 50% by weight of ethers and from 10 to 40% by weight of alcohols.

The ethers are typical dialkyl ethers and at least one of the alkyl group is usually branched.

The liquid product from step (a) can be added to any gasoline for example a straight run gasoline, a cat cracked spirit or mixtures thereof. Preferably the gasoline has, before the addition of the additive composition a Research Octane Number (RON) of from 80 to 105, more preferably 90 to 98, and a Motor Octane Number (MON) of from 75 to 95, more preferably from 80 to 88. The gasoline may contain the following proportions of olefins, aromatics and saturates:

Aromatics 20 to 60% volume
Saturates 20 to 70% volume
Olefins 0 to 30% volume

By the term liquid product we mean a product which is liquid at 20°C and one atmosphere pressure.

The liquid product from step (a) possesses high Blending Research Octane Number and Blending Motor Octane Number which generally increase with increasing amounts of oxygenated components. Typical values are in the range 100 to 116 for Research Octane Number and 95 to 106 for Motor Octane Number in one gasoline basestock. The Blending Octane Numbers obtained will vary with the composition of the gasoline used as basestock.

According to one embodiment of the invention the etherification step (b) precedes step (a) and olefinic hydrocarbons depleted in branched olefins are passed to step (a) from step (b).

Preferably according to this embodiment the $C_3$ to $C_6$ alkenes contain isobutene and the isobutene is reacted with methanol to form methyl tertiary butyl ether in step (b) and the olefinic hydrocarbons depleted in isobutene passed from step (b) to step (a).

By the term branched olefin or branched alkene we mean an olefin or alkene which contains branching at a carbon atom bearing a double bond i.e. contains the structure

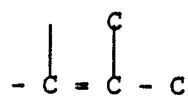

The catalyst for step (b) is preferably the same as that for step (a). Conveniently the $C_1$ to $C_6$ alcohol is methanol or a higher alcohol such as ethanol, propanol, butanol or the like and is added to the product from step (a) at a point intermediate steps (a) and (b). The conditions for the etherification step (b) can be:-

Temperature 50 to 130°C, preferably 70 to 100°C

Methanol content of feed 0.1 to 100 vol % of product from step (a) preferably 10 to 40 vol %.

LHSV from 1 to 10 preferably 2 to 5.

The feed to the alkylation step contains $C_4$ alkenes optionally together with $C_3$ alkenes and/or $C_5$ alkenes.

The alkylation step (c) can be carried out under the following conditions:

temperature from 10 to 50°C

pressure from 5 to 40 bar

The catalyst is conveniently hydrofluoric acid or sulphuric acid. In one embodiment of the process the feed contains isobutane and the steps (a) and (b) are controlled so that the molar ratio of isobutane to $C_3$ to $C_5$ alkenes in the product from the second step (after removal of liquid product) is in the range 1:2 to 2:1 and this product is passed to step (c).

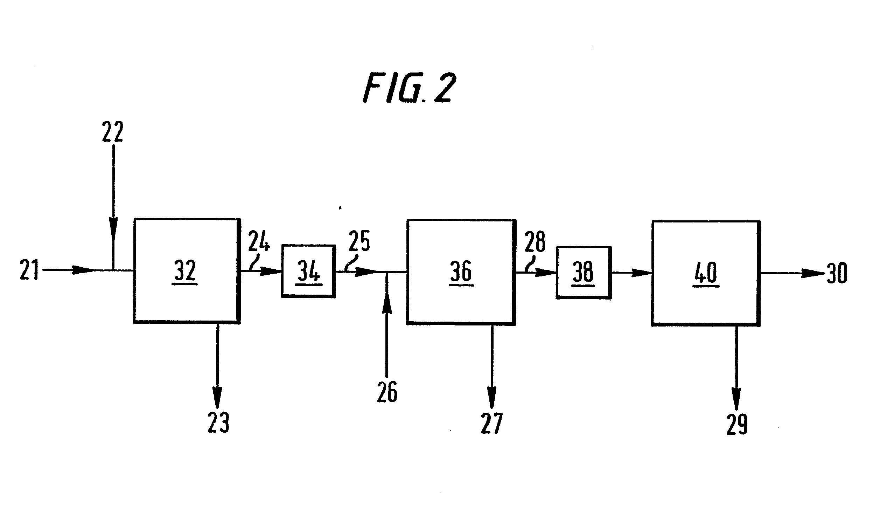

The invention is illustrated by reference to the accompanying drawings in which Figure 1 illustrates one mode of operation in which three steps comprising (a) oligomerisation/etherification/hydration, (b) etherification and (c) alkylation are carried out in that order and Figure 2 illustrates an alternative mode of operation in which the etherification step precedes the oligomerisation/etherification hydration step. Referring to Figure 1 a feed from a cat. cracker containing $C_3$ to $C_5$ olefins from which butadiene and the bulk of the isobutene have been consumed by other processes and having the composition set forth in Table 1 is passed with added water through line 2 to the hydration/etherification/oligomerisation unit 4. The unit 4 contains a bed of Rohm & Haas XE-386 ion exchange resin. The alkene:water molar ratio is 42:1. The temperature in the bed ranges from 126°C to 146°C at the end of the bed. The reaction pressure is 80 bar and the LHSV is 2. Under these conditions 42.5% of the alkenes are converted to liquid products. The degassed liquid product contains about 96% wt oligomers of butenes (with a $C_8$:$C_{12}$ alkene weight ratio of 20:1 and no higher oligomers than $C_{12}$), and 4.0% oxygenates (1.2% secondary butanol and 2.8% disecondary-butyl ether). No water is detected in the liquid product. In one mode of operation unreacted gases which comprise mainly $C_3$, $C_4$ and $C_5$ alkenes and alkanes are separated from the liquid product and passed to alkylation unit 16 via line 13. In an alternative mode of operation the unreacted gases are passed with the liquid product via line 6 to etherification unit 8. In either mode the liquid product from the unit 4 together with methanol added via line 7 pass via line 6 to the etherification unit 8 where the oligomers (particularly oligomers of $C_4$ alkenes containing a tertiary carbon atom with a double bond) are reacted with the added methanol to form the corresponding ethers. The liquid reaction product is withdrawn via line 12. $C_3$ and $C_4$ alkenes are separated from the liquid product and passed together with added isobutane to an alkylation unit 16 via line 15.

Methanol can be removed from the liquid product from unit 8 and recycled via line 14.

A guard bed (not shown) situated immediately before the alkylation unit removes any oxygen-containing compounds.

Table 1

| Component | % weight |
|---|---|
| n butane | 22.7 |
| isobutane | 4.4 |
| butene-1 | 55.1 |
| butene-2 | 16.3 |
| isobutene | 1.3 |
| pentanes | 0.1 |

The feed to the etherification unit 8 has a molar ratio of oligomers:methanol of 2:1.

The conditions in the etherification unit are as follows: temperature of the bed 80 to 85°C at end of bed
pressure 80 bar
space velocity 2.

The catalyst consists of a bed of Rohm & Haas XE-386 ion exchange resin. Under the above conditions 5% of the oligomers are converted to the corresponding methyl ethers and withdrawn via line 12 together with other unreacted product from unit 4. The B MON of the degassed product is increased by 4 B MON in comparison with the B MON of product from unit 4.

In one of the modes of operation referred to above $C_3$, $C_4$ and $C_5$ alkenes and alkanes are separated from the product from unit 4 and passed via line 13 to the alkylation unit 16. In this case the effluent from unit 8, contains methanol and liquid etherified product. The methanol can be separated and recycled via line 14 to the feed to unit 8. There therefore need be no direct connection between units 8 and 16 in this mode of operation.

Isobutane is added via line 17 to give a molar ratio of isobutane to total olefins ($C_3$, $C_4$ and $C_5$ alkenes) of 1:1. The conditions in the alkylation unit 16 are as follows:
temperature 35°C
pressure 30 bar
catalyst HF added continuously with recycle.

The composition of the alkylate is found to be at least 80% eg 95% of $C_8$ branched alkanes and $C_4$ byproduct (mainly n butane).

The advantages of the above described combination of processes are:

(1) the effect of step (a) is to increase the molar ratio of isobutane/olefin and also to increase the molar ratio of butene-2 to butene-1 from 0.3 to 1 which is a typical figure for a $C_4$ stream from a steam cracker to about 4.5 to 1 which is typical for the gas stream from step (a).

(2) the effect of step (b) is to increase the octane no of the liquid product of step (a) and also to reduce the olefin content of the liquid product of step (a).

The invention is illustrated by the following Examples. Example 1 is an example of both steps (a) and (b) of the process of claim 1. Example 2 is an example of step (b) of the process of claim 1.

## Example 1

A butanes/butenes feedstock, containing 63.7% alkenes of composition shown in Table 2 and water in the alkenes:water molar ratio of 40:1 were passed through a two stage two reactor system with an overall LHSV of 2 at a reaction pressure of 80 bar. The first reactor contained 30 ml of dried Amberlite XE-386, a strongly acidic resin catalyst produced by the Rohm and Haas Company, maintained at a temperature of ca 130 to 140°C. The cooled products from the first reactor, together with added methanol (in an amount corresponding to a molar ratio of methanol to alkenes in the feedstock entering the first reactor of 1:10) were passed through a second reactor containing 30 ml of dried Amberlite XE-386 maintained at a reaction temperature of ca 80-85°C.

The products leaving the second reactor are cooled and pass through a pressure relief valve to the liquid product collecting pot at room temperature. It is known that some products are lost in the gases passing to vent. Warming the liquid products to 65°C to remove dissolved feedstock gave liquid products corresponding to an alkenes conversion of 40%; the analysis of which is shown in the Table. The products contain some methoxyoctanes which probably increase the octane quality of the products.

Example 2

A feedstock consisting of hydrocarbons and a small amount of oxygenates, prepared by oligomerising and hydrating a $C_3/C_4$ catalytic cracker stream as described in European Patent Application No 196902 and with the analysis shown in Table 2 and methanol were combined in the weight ratio of 12:1 and passed over 30 ml of dried Amberlite XE-386 with an LHSV of 2 at a reaction temperature of 73-85°C and reaction pressure of 30 bar. The products passed through a pressure relief valve and the liquid products collected at atmospheric pressure. The major hydrocarbon products had a composition shown in the Table, and contained significant amounts of methoxyalkanes of carbon numbers of $C_6$ to $C_{10}$ which should increase the octane quality of the products. A second very minor product phase (<1%) consisting of 85% methanol and 15% water was also obtained which could be recycled if desired.

Table 2

Composition of Reactant and Product Streams (% Weight)

| Component | Feedstock for Example 1 | Products leaving 1st reactor in Example 1 | Product from Example 1 | Feedstock for Example 2 | Product from Example 2 |
|---|---|---|---|---|---|
| Propane | 0.2 | 0.2 | - | 1.1) | 0.3) |
| Propene | | | - | ) | ) |
| Butane | 30.0 | 29.9 | ) | ) | ) |
| Isobutane | 6.1 | 6.0 | ) | ) | ) |
| But-2-ene | 18.9 | 29.6 | 7.0) | 3.9) | 2.2) |
| But-1-ene | 43.4 | 4.9 | ) | ) | ) |
| Isobutene | 1.4 | 0 | ) | ) | ) |
| $C_5$-$C_7$ Alkenes | | 0.2 | 0.7 | 23.9 | 18.8 |
| $C_8$ Alkenes | | 24.0 | 73.8 | 10.0 | 10.5 |
| $C_{9-11}$ Alkenes | | 1.1 | 3.6 | 51.3 | 52.1 |
| $C_{12}$ Alkenes | | 1.2 | 4.2 | 5.8 | 6.2 |
| $>C_{12}$ Alkenes | | 0.1 | 0.4 | 2.5 | 2.5 |
| Methanol | | | 0.1) | - | 2.0 |
| Dimethyl Ether | | | ) | - | 0.1 |
| Isopropanol | | | - | 0.1 | 0.1 |
| Diisopropyl Ether | | | - | 0.8 | 0.8 |
| Sec-Butanol | | 1.2 | 1.5 | 0.4 | 0.3 |
| Isopropyl sec-butyl Ether | | | - | 0.2 | 0.2 |
| Di-sec-butyl Ether | | 1.6 | 4.7 | - | - |
| 2-Methoxybutane | | | 2.5 | - | - |
| Methoxyalkanes ($C_6$-$C_{10}$) | | | 1.5 | - | 3.8 |

Example 3

In the mode of operation shown in Figure 2 (which is a 3 step process involving (i) etherification to form methyltertiarybutyl ether; (ii) oligomerisation/hydration/etherification and (iii) alkylation) a $C_4$ cat. cracker stream 21 of composition shown in Table 3 is passed with added methanol 22 to an etherification unit 32 where the methanol is reacted with the isobutene to form methyl tertiary butyl ether which is withdrawn as stream 23. Unreacted materials together with any byproducts leave unit 32 as stream 24 and are passed through a methanol remover 34 and then as stream 25 to which a water stream 26 is added and passed to unit 36 where the alkenes are oligomerised/hydrated/etherified to form a liquid product which is withdrawn as stream 27. Unreacted materials and byproducts are passed as stream 28 via guard bed 38 to remove oxygenates to alkylation unit 40 where isobutane is reacted with $C_4$ olefins to produce $C_8$ isoalkanes which are withdrawn as stream 29. Unreacted n-butane is withdrawn as stream 30.

Table 3

| | |
|---|---|
| n-butane | 14.3 |
| i-butane | 24.4 |
| but-1-ene | 15.2 |
| but-2-ene | 27.5 |
| isobutene | 18.6 |

The process shown in Figure 2 can be run continuously and Table 4 indicates the composition of the various streams and their amounts in tonnes for 1 year of continuous operation.

Table 4

Figures in tonnes per annum

| | 21 | 22 | 23 | 24 | 25 | 26 | 27 | 28 | 29 | 30 |
|---|---|---|---|---|---|---|---|---|---|---|
| n-butane | 50900 | | | 50900 | 50900 | | | 50900 | | 50900 |
| isobutane | 86900 | | | 86900 | 86900 | | | 86900 | | |
| but-1-ene | 54100 | | | 52000 | 52000 | | | 122000 | | |
| bute-2-ene | 97900 | | | 100000 | 100000 | | | 71700 | | |
| isobutene | 66200 | | | 3200 | 3200 | | | | | |
| water | | | | | | 1200 | | | | |
| methanol | | 37700 | | 1800 | | | | | | |
| etherified product | | | | | | | 72500 | | | |
| alkylate | | | | | | | | | 171200 | |
| MTBE | | | 98900 | | | | | | | |
| TOTAL | 356000 | 37700 | 98900 | 294800 | 293000 | 1200 | 72500 | 221700 | 171200 | 50900 |

Total Liquid Product of High Octane 342,600

## Claims

1. A process for the production of gasoline range products from $C_3$ to $C_6$ alkenes which process comprises the following steps:

(a) passing a feed containing the $C_3$ to $C_6$ alkenes and water in a molar ratio of alkene to water of from 3:1 to 90:1 over an acid hydration catalyst under hydration conditions to form a product containing oligomers of the alkenes, ethers and optionally alcohols and

(b) an etherification step in which alkenes containing branching at a carbon atom bearing a double bond are reacted with a $C_1$ to $C_6$ alcohol to form ethers.

2. A process as claimed in claim 1 wherein in step (a) the molar ratio of alkene to water is from 4:1 to 50:1, the temperature is within the range 120 to 180°C, the pressure from 20 to 100 bar absolute and the LHSV of feed from 0.5 to 10.

3. A process as claimed in claim 1 or 2 wherein step (a) precedes step (b) and the $C_3$ to $C_6$ alkenes from step (a) are passed to step (b).

4. A process for the production of gasoline range products from $C_3$ to $C_5$ alkenes which process comprises the following steps:

(a) passing a feed containing a mixture of alkenes and alkanes, at least 95% by weight of which feed is of carbon number $C_3$ to $C_5$ and at least 50% by weight of the feed is alkenes with water in a molar ratio of alkene to water of 3:1 to 90:1 over an acid hydration catalyst under hydration conditions to form a product containing oligomers of the alkenes, ethers and optionally alcohols,

(b) reacting branched alkenes from step (a) with methanol in an etherification step under etherification conditions to form ethers.

5. A process as claimed in claim 4 wherein $C_3$ to $C_5$ alkenes from step (b) are passed to an alkylation step (c) where they are reacted with isobutane to form $C_7$ to $C_9$ alkylate.

6. A process as claimed in claim 4 wherein $C_3$ to $C_5$ alkenes from step (a) are passed directly to the alkylation step (c).

7. A process as claimed in claim 1 wherein step (b) precedes step (a) and the process comprises reacting the feed containing the $C_3$ to $C_6$ alkenes with the $C_1$ to $C_6$ alcohol in an etherification stage under etherification conditions so that branched alkenes react with the alcohol to form ethers and passing $C_3$ to $C_6$ alkenes depleted in branched alkenes from the etherification step to the hydration step (a) to form ethers, oligomers and optionally alcohols.

8. A process as claimed in claim 6 which comprises passing $C_3$ to $C_5$ alkenes from step (a) to an alkylation step (c) where they are reacted wiith isobutane to form $C_7$ to $C_9$ alkylate.

FIG. 1

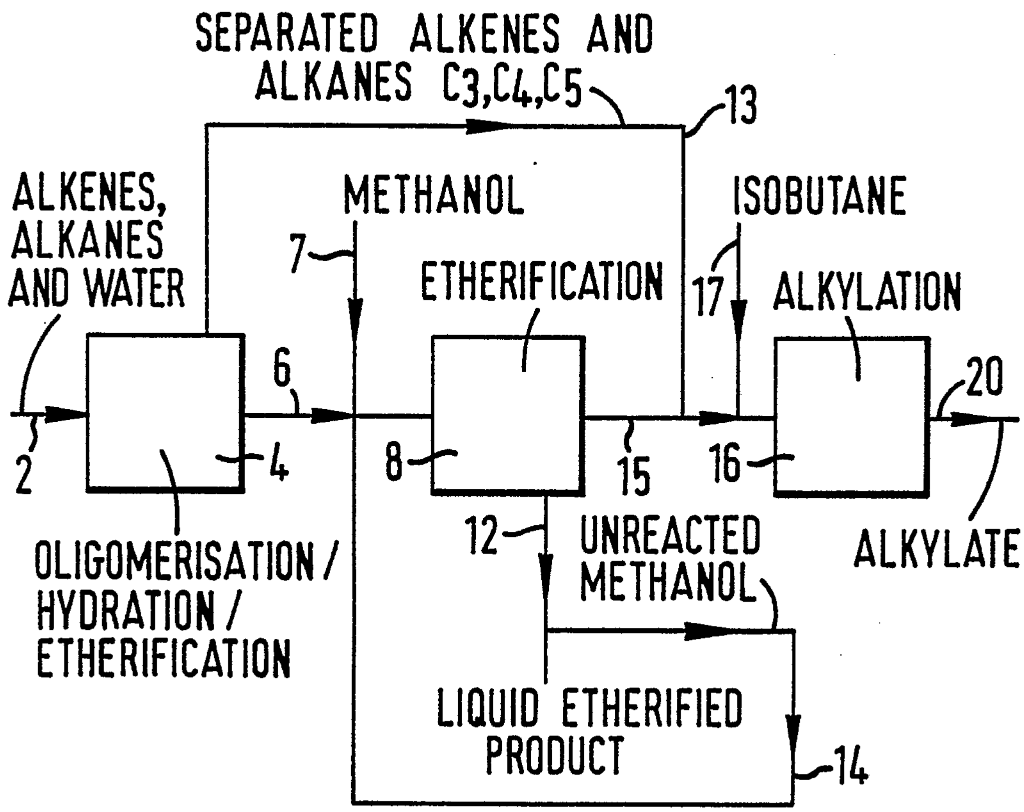


In one mode of operation.

| | | | | |
|---|---|---|---|---|
| Stream (2) | 220,000 t/a | Stream (15) | | |
| Water | 2,200 t/a | | $C_4$ | 154,500 t/a |
| Stream (6) | 67,700 t/a | Stream (17) | | 72,800 t/a |
| $C_4$ | 154,500 t/a | | | Isobutane |
| Stream (7) | 9,026 t/a | Stream (20) | | Alkylate |
| Methanol | | | | 171,200 t/a |

Stream (12)
Liquid + Methanol 76,726 t/a

FIG. 2